Europäisches Patentamt

European Patent Office

Office européen des brevets

(19)

(11) Veröffentlichungsnummer: **0 313 773**
**A1**

(12) **EUROPÄISCHE PATENTANMELDUNG**

(21) Anmeldenummer: **88114493.5**

(22) Anmeldetag: **06.09.88**

(51) Int. Cl.4: **A61F 2/34**

(30) Priorität: **28.10.87 CH 4226/87**

(43) Veröffentlichungstag der Anmeldung:
**03.05.89 Patentblatt 89/18**

(84) Benannte Vertragsstaaten:
**AT DE FR GB IT**

(71) Anmelder: **GEBRÜDER SULZER AKTIENGESELLSCHAFT**
**Zürcherstrasse 9**
**CH-8401 Winterthur(CH)**

Anmelder: **PROTEK AG**
**Stadtbachstrasse 64**
**CH-3003 Bern(CH)**

(72) Erfinder: **Müller, Maurice, Prof. Dr.-med.**
**Melchenbühlweg 9**
**CH-3006 Bern(CH)**

(74) Vertreter: **Dipl.-Ing. H. Marsch Dipl.-Ing. K. Sparing Dipl.-Phys.Dr. W.H. Röhl**
**Patentanwälte**
**Rethelstrasse 123**
**D-4000 Düsseldorf 1(DE)**

(54) **Künstliche Hüftgelenkspfanne.**

(57) Der Pfannenkörper (6) trägt im Bereich des Aequators einen Kranz von Vorsprüngen (4), deren Achsen auf dem Umfang in gleichen Winkel-"Abständen" verteilt sind. Diese Vorsprünge (4) greifen in Nuten (3) im Rand (2) der Aussenschale (1) ein. Beide Elemente (1 und 6) sind durch einen Schnappverschluss relativ zueinander fixiert.

Die Nuten (3) und die Vorsprünge (4) bilden eine intraoperativ einstellbare Verdrehsicherung zwischen den beiden Teilen (1) und (6) der Hüftgelenkspfanne, so dass die Lage einer Unsymmetrie des Pfannenkörpers (6) während der Operation relativ zu einem Bezugspunkt verändert werden kann.

Fig.1

## Künstliche Hüftgelenkspfanne

Die Erfindung betrifft eine künstliche Hüftgelenkspfanne, bestehend aus einer metallenen Aussenschale und aus einem, die Pfannenschale für die Aufnahme eines Gelenkkopfes enthaltenden Kunststoff-Pfannenkörper, der in der Aussenschale fixierbar ist, wobei der Pfannenkörper in Bezug auf die Scheitelachse der Aussenschale unsymmetrisch ausgebildet ist.

Hüftgelenkspfannen der genannten Art sind beispielsweise aus der CH-Patentanmeldung 1219/87-0 bekannt; bei ihnen muss der Pfannenkörper mit einer Rotationssicherung gegenüber der Aussenschale versehen werden. Ist der Pfannenkörper nun bezüglich der Rotations-Symmetrieachse der Aussenschale bzw. der Pfannenschale unsymmetrisch ausgebildet, so ist es häufig erforderlich, dass die Winkellage dieser Unsymmetrie relativ zu einem Bezugspunkt im Körper bei schon implantierter Aussenschale intraoperativ korrigiert werden muss.

Aufgabe der Erfindung ist es daher, eine zweiteilige Hüftgelenkspfanne zu schaffen, bei der die Winkellage des Pfannenkörpers gegenüber der Aussenschale während der Operation verändert werden kann und der Pfannenkörper gegenüber der Aussenschale gegen unbeabsichtigte Rotationen gesichert ist.

Mit der Erfindung wird diese Aufgabe dadurch gelöst, dass der Umfang des Pfannenkörpers im Bereich des Aequators mindestens teilweise mit einer Reihe einzelner, kranzartig angeordneter Vorsprünge versehen ist, deren Achsen um einen auf mindestens auf einem Teil des Umfanges konstanten Winkelbetrag gegeneinander versetzt sind, und dass ferner der äquatoriale Rand der Aussenschale auf mindestens einem Teil seines Umfanges mit an die Vorsprünge angepassten Nuten ausgestattet ist.

Aufgrund der kranzartig angeordneten Vorsprünge, die vorteilhafterweise jeweils nach einer Drehung von $\pi/8$ in Umfangsrichtung in die Aussenschale einrasten, kann die Lage der Unsymmetrie des Pfannenkörpers relativ zu einem Bezugspunkt noch während der Operation eingestellt und verändert werden.

Aussenschale und Pfannenkörper sind aus in der Implantat-Technik üblichen Werkstoffen; so besteht die Aussenschale beispielsweise aus Reintitan oder einer Titanlegierung, während der Pfannenkörper aus Polyäthylen gefertigt ist.

Im folgenden wird die Erfindung anhand eines Ausführungsbeispiels im Zusammenhang mit der Zeichnung näher erläutert.

Fig. 1 zeigt eine Aufsicht auf die Hüftgelenkspfanne vom Aequator her in Richtung der Scheitelachse;

Fig. 2 ist eine Ansicht von Fig. 1 von rechts, teilweise im Schnitt dargestellt;

Fig. 3 ist ein Detail aus Fig. 2 in vergrössertem Massstab.

Die stark schematisiert dargestellte halbkugelförmige Aussenschale 1 ist auf ihrem äquatorialen Rand 2 mit Nuten 3 versehen, die auf dem Umfang des Randes 2 regelmässig verteilt sind, wobei die Teilung mindestens über einen Teil des Umfanges in Schritten von $\pi/8$ erfolgt ist, selbstverständlich sind jedoch auch andere Teilungen, beispielsweise $\pi/6$ oder $20°$, möglich.

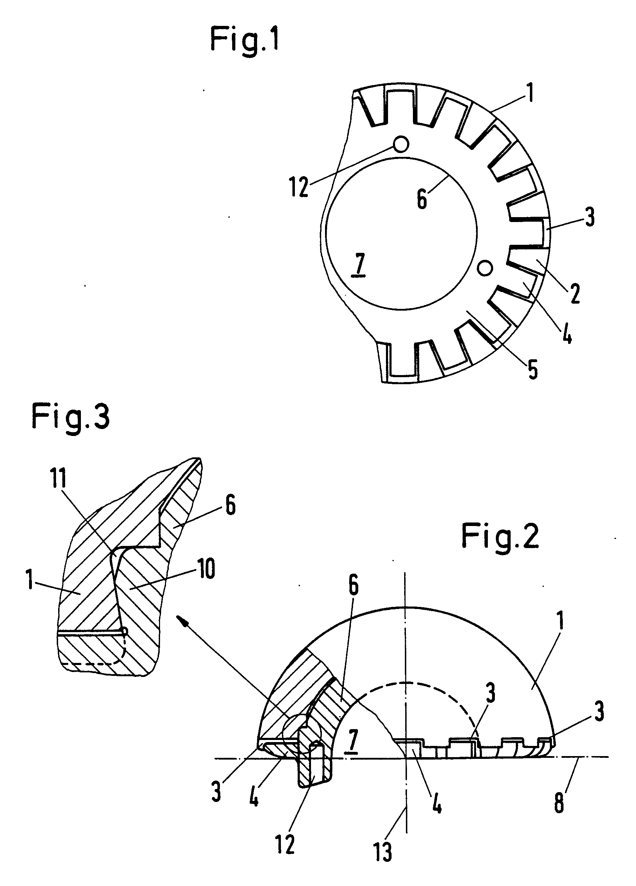

In diesen Nuten 3 eingebettet sind Vorsprünge 4, die den äquatorialen Rand 5 des Pfannenkörpes 6 umgeben; dieser enthält die eigentliche Pfannenschale 7 zur Aufnahme eines nicht gezeigten Gelenkkopfes und ist auf einem Teil seines Umfanges über die Aequatorebene 8 hinaus verlängert, also bezüglich der Scheitelachse 13 (Fig. 2) unsymmetrisch ausgebildet.

Die Fixierung des Pfannenkörpers 6 in der Aussenschale 1 erfolgt durch einen Schnappverschluss, der aus einem ringförmigen Ansatz 10 (Fig. 3) an der äusseren Kugelfläche des Pfannenkörpers 6 und einer entsprechenden Ausnehmung 11 im Innenraum der Aussenschale 1 besteht.

Wie Fig. 3 zeigt, sind die Elemente 10 und 11 des Schnappverschlusses mit sich in Einpressrichtung des Pfannenkörpers 6 erweiternden konischen Flanken ausgestattet, durch die ein fester Sitz des Pfannenkörpers 6 in der Aussenschale gewährleistet wird.

Im Bund 5 des Pfannenkörpers 6 sind Bohrungen 12 für den Eingriff eines Verdreh- und Einpressinstrumentes vorgesehen.

## Ansprüche

1. Künstliche Hüftgelenkspfanne bestehend aus einer metallenen Aussenschale und aus einem, die Pfannenschale für die Aufnahme eines Gelenkkopfes enthaltenden Kunststoff-Pfannenkörper, der in der Aussenschale fixierbar ist, wobei der Pfannenkörper in Bezug auf die Scheitelachse der Aussenschale unsymmetrisch ausgebildet ist, **dadurch gekennzeichnet, dass** der Umfang des Pfannenkörpers (6) im Bereich des Äquators mindestens teilweise mit einer Reihe einzelner, kranzartig angeordneter Vorsprünge (4) versehen ist, deren Achsen um einen auf mindestens auf einem Teil des Umfanges konstanten Winkelbetrag gegeneinander versetzt sind, **und dass ferner** der äquatoriale Rand (2) der Aussenschale (1) auf mindestens einem Teil seines Umfanges mit an die Vorsprünge (4) angepassten Nuten (3) ausgestattet ist.

2. Hüftgelenkspfanne nach Anspruch 1, **dadurch gekennzeichnet, dass** der Winkel zwischen den Achsen der Vorsprünge (4) $\pi/8$ beträgt.

# Fig.1

# Fig.3

# Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-2 950 536 (HOWMEDICA) <br> * Figuren 1,2,4,5; Ansprüche 1,9 * | 1 | A 61 F 2/34 |
| A | | 2 | |
| | --- | | |
| Y | US-A-4 695 282 (M. FORTE et al.) <br> * Figur 1; Zusammenfassung * | 1 | |
| | ----- | | |

**RECHERCHIERTE SACHGEBIETE (Int. Cl.4)**

A 61 F

Der vorliegende Recherchenbericht wurde für alle Patentansprüche erstellt

| Recherchenort | Abschlußdatum der Recherche | Prüfer |
|---|---|---|
| DEN HAAG | 29-12-1988 | NEILL M.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P0403)